# EUROPEAN PATENT APPLICATION

(11) **EP 4 458 395 A1**
(43) Date of publication of application: **06.11.2024**
(21) Application number: 21969782.8
(22) Date of filing: 31.12.2021
(51) Int. Cl.: A61M 16/01, A61M 5/172, A61B 5/00

(54) **ANESTHESIA CONTROL SYSTEM, ANESTHESIA SYSTEM, AND NON-TRANSITORY COMPUTER READABLE MEDIUM**

(71) Applicant: GE Precision Healthcare LLC, Milwaukee, WI 53226 (US)
(72) Inventor: SONG, Min, Shanghai 201203 (CN); WU, Kaikai, Shanghai 201203 (CN)
(74) Representative: Kilburn & Strode LLP
(86) International application number: PCT/CN2021/143896
(87) International publication number: WO 2023/123437

(57) **Abstract**

The present application provides an anesthesia control system configured to: acquire a plurality of physiological parameters of a patient; acquire anesthetic parameters of a plurality of anesthesia devices applied to the patient; acquire a depth of anesthesia index of the patient; and automatically generate a control instruction based on the plurality of physiological parameters, the anesthetic parameters, the depth of anesthesia index, and a target depth of anesthesia index, the control instruction being used to control the anesthetic parameters of each of the plurality of anesthesia devices such that the depth of anesthesia index of the patient is regulated to the target depth of anesthesia index. The present application further provides an anesthesia system and a non-transitory computer-readable medium.

## Description

### TECHNICAL FIELD

The present application relates to anesthesia control, and more particularly, relates to an anesthesia control system, an anesthesia system and a non-transitory computer readable medium.

### BACKGROUND

Anesthesia is a widely used medical procedure. For example, during a surgical operation, a doctor can suppress a patient's central nervous system by anesthetizing the patient, thereby creating a surgical condition and eliminating the patient's pain and conscious discomfort.

Anesthesia may include inhalation anesthesia and injection anesthesia. The former will produce an anesthetic effect by causing a patient to inhale a vaporized anesthetic. The latter will produce an anesthetic effect by administering an anesthetic to a patient through intravenous or blood injection. Different anesthetics are used in different anesthesia techniques, and the anesthetic effects are different. Clinically, a patient can be subjected to mixed anesthesia using different anesthesia techniques at the same time, thereby achieving a good anesthetic effect.

A plurality of different anesthetics are used in the mixed anesthesia, and different anesthetics may have the same anesthetic action. In addition, the anesthetic actions of different anesthetics may affect each other. In addition, there may be an antagonistic action between different anesthetics. Therefore, regulation on the anesthetic dosage in the mixed anesthesia becomes very complicated. On the one hand, when the dosage of a certain anesthetic is regulated, it is necessary to pay attention to all physiological indicators of a patient at the same time. On the other hand, it is also necessary to consider whether to regulate the dosages of other anesthetics. This is a test of the professional skill and experience of the anesthetist.

### SUMMARY

The aforementioned defects, deficiencies, and problems are solved herein, and these problems and solutions will be understood through reading and understanding the following description.

Some embodiments of the present application provide an anesthesia control system, the anesthesia control system being configured to: acquire a plurality of physiological parameters of a patient; acquire anesthetic parameters of a plurality of anesthesia devices applied to the patient; acquire a depth of anesthesia index of the patient; and automatically generate a control instruction based on the plurality of physiological parameters, the anesthetic parameters, the depth of anesthesia index, and a target depth of anesthesia index, the control instruction being used to control the anesthetic parameters of each of the plurality of anesthesia devices such that the depth of anesthesia index of the patient is regulated to the target depth of anesthesia index.

Some embodiments of the present application provide an anesthesia system, comprising: a physiological parameter acquisition apparatus used to acquire a plurality of physiological parameters of a patient; a plurality of anesthesia devices used to administer an anesthetic to the patient; a depth of anesthesia index acquisition apparatus used to acquire a depth of anesthesia index of the patient; and an anesthesia control system connected to the physiological parameter acquisition apparatus, the plurality of anesthesia devices and the depth of anesthesia index acquisition apparatus. The anesthesia control system is configured to: acquire a plurality of physiological parameters of a patient; acquire anesthetic parameters of a plurality of anesthesia devices applied to the patient; acquire a depth of anesthesia index of the patient; and automatically generate a control instruction based on the plurality of physiological parameters, the anesthetic parameters, the depth of anesthesia index, and a target depth of anesthesia index, the control instruction being used to control the anesthetic parameters of each of the plurality of anesthesia devices such that the depth of anesthesia index of the patient is regulated to the target depth of anesthesia index.

Some embodiments of the present application provide a non-transitory computer-readable medium, the non-transitory computer-readable medium storing a computer program having at least one code segment that is executable by a machine to: acquire a plurality of physiological parameters of a patient; acquire anesthetic parameters of a plurality of anesthesia devices applied to the patient; acquire a depth of anesthesia index of the patient; and automatically generate a control instruction based on the plurality of physiological parameters, the anesthetic parameters, the depth of anesthesia index, and a target depth of anesthesia index, the control instruction being used to control the anesthetic parameters of each of the plurality of anesthesia devices such that the depth of anesthesia index of the patient is regulated to the target depth of anesthesia index.

It should be understood that the brief description above is provided to introduce, in a simplified form, concepts that will be further described in the detailed description. The brief description above is not meant to identify key or essential features of the claimed subject matter. The scope is defined uniquely by the claims that follow the detailed description. Furthermore, the claimed subject matter is not limited to implementations that solve any deficiencies raised above or in any section of the present disclosure.

### BRIEF DESCRIPTION OF THE DRAWINGS

The present invention will be better understood by reading the following description of non-limiting examples with reference to the accompanying drawings, wherein
FIG. 1 is a schematic diagram of an anesthesia system according to some embodiments of the present application;
FIG. 2 is a control method flowchart of an anesthesia control system according to some embodiments of the present application;

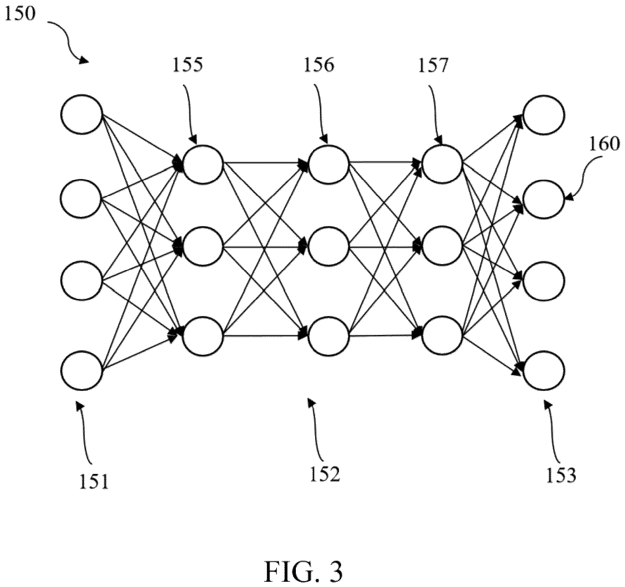

FIG. 3 is a diagram of a learning network according to some embodiments of the present application; and
FIG. 4 is a control method flowchart of an anesthesia control system according to some additional embodiments of the present application.

### DETAILED DESCRIPTION

Specific embodiments of the present disclosure will be described below, but it should be noted that in the specific description of these embodiments, for the sake of brief description, it is impossible to describe all features of the actual embodiments of the present disclosure in detail. It should be understood that in the actual implementation process of any embodiment, just as in the process of any one engineering project or design project, a variety of specific decisions are often made to achieve specific goals of the developer and to meet system-related or business-related constraints, which may also vary from one embodiment to another. Furthermore, it should also be understood that although efforts made in such development processes may be complex and tedious, for a person of ordinary skill in the art related to the content disclosed in the present disclosure, some design, manufacture, or production changes made on the basis of the technical content disclosed in the present disclosure are only common technical means, and should not be construed as the content of the present disclosure being insufficient.

Unless otherwise defined, the technical or scientific terms used in the claims and the description should be as they are usually understood by those possessing ordinary skill in the technical field to which they belong. The terms "first", "second", and similar ones used in the present disclosure and the claims do not imply any order, quantity, or importance, but are merely intended to distinguish between different components. The terms "one" or "a/an" and similar terms do not express a limitation of quantity, but rather that at least one is present. The terms "include" or "comprise" and similar words indicate that an element or object preceding the terms "include" or "comprise" encompasses elements or objects and equivalent elements thereof listed after the terms "include" or "comprise", and do not exclude other elements or objects. The terms "connect" or "link" and similar words are not limited to physical or mechanical connections, and are not limited to direct or indirect connections.

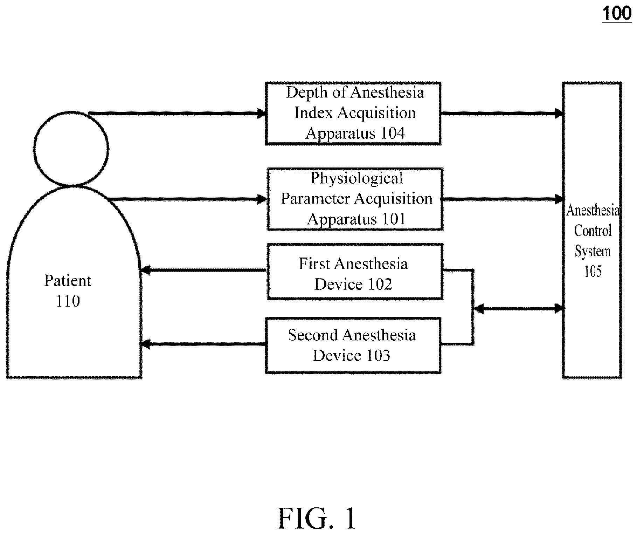

Referring to FIG. 1, FIG. 1 is a schematic diagram of an anesthesia system 100 according to some embodiments of the present application.

The anesthesia system 100 includes: a physiological parameter acquisition apparatus 101 used to acquire a plurality of physiological parameters of a patient 110; a plurality of anesthesia devices including a first anesthesia device 102 and a second anesthesia device 103, said devices being used to administer an anesthetic to the patient 110; a depth of anesthesia index acquisition apparatus 104 used to acquire a depth of anesthesia index of the patient; and an anesthesia control system 105 connected to the physiological parameter acquisition apparatus 101, the plurality of anesthesia devices and the depth of anesthesia index acquisition apparatus 104. Individual components of the anesthesia system 100 are exemplarily described below.

The type of the physiological parameter acquisition apparatus 101 may be diverse. For example, it may be any one or both of a monitor and an electrocardiograph. The physiological parameter acquisition apparatus 101 may be used for conventional physiological parameter measurement of the patient 110. In one example, the physiological parameter acquisition apparatus 101 includes a variety of different measurement modules, such as several among a heart rate measurement module, an oxygen saturation measurement module, a blood pressure measurement module, a blood glucose measurement module, an end-tidal carbon dioxide concentration measurement module, and an end-tidal oxygen concentration measurement module. These different modules may be integrated in the same instrument, or may be modules distributed in different medical devices, and can be freely selected by doctors as needed.

The plurality of anesthesia devices may include the first anesthesia device 102 and the second anesthesia device 103. In one example, the first anesthesia device 102 is different from the second anesthesia device 103. For example, the first anesthesia device 102 may be an inhalation anesthesia device, and the second anesthesia device 103 may be an injection anesthesia device. Specific configurations of the inhalation anesthesia device and the injection anesthesia device may be arbitrary in the art, and are described only as an example in the present application.

The inhalation anesthesia device may include an anesthesia machine or an anesthetic and an associated gas supply system thereof. In one example, the gas supply system may supply gas to the anesthetic through a conduit. The type of the gas includes, but is not limited to, air, oxygen, and nitrous oxide. The anesthesia machine contains a vaporizer used to vaporize an anesthetic. The gas in the gas supply system, after entering the anesthesia machine, can be mixed with the vaporized anesthetic and then provided to the patient. Anesthetic parameters of the inhalation anesthesia device can be regulated. For example, during use of the anesthetic vaporizer, the dosage of anesthetic supplied to the patient may be regulated by regulating the flow rate of the gas from the gas supply system to the vaporizer. The specific manner of regulation may be implemented by a plurality of flow regulating apparatuses (e.g., valves). In addition, the amount of the vaporized anesthetic may be controlled by controlling the vaporizer of the anesthesia machine. With such a configuration, regulation of the anesthetic parameters of the anesthetic supplied to the patient can be achieved. In addition, the inhalation anesthesia device may further include a module such as a sensor for monitoring the state of the anesthesia machine or the state of the patient, which will not be described in detail.

The injection anesthesia device may include an anesthetic injection module and a control module. In one example, a plurality of anesthetic injection modules may be included, and each of the plurality of anesthetic injection modules may be used to inject anesthetics having different effects. Each anesthetic injection module may include a syringe pump. The specific configuration of the syringe pump may be arbitrary in the art, and will not be described in detail. The control module may obtain the current injection rate and/or an anesthetic dosage of the syringe pump by calculation or the like, thereby controlling the injection rate of the syringe pump.

It is to be understood that the above description on the inhalation anesthesia device and the injection anesthesia device is merely illustrative and not exclusively restrictive. The specific configurations of the devices may be arbitrary in the prior art, and will not be enumerated one by one.

The depth of anesthesia index acquisition apparatus 104 is used to acquire a depth of anesthesia index of a patient. The depth of anesthesia index may adopt common indexes in the art, for example, the depth of anesthesia index may include at least one of a sedation index, a pain threshold index, and a muscle relaxation index. In some examples, the depth of anesthesia index includes all three among a sedation index, a pain threshold index, and a muscle relaxation index. The manners of acquisition and determination of the above three indexes may be varied. For example, the depth of anesthesia index acquisition apparatus 104 may include an electroencephalograph (EEG) for acquiring a reaction entropy (RE) and a state entropy (SE), where the reaction entropy can be obtained by analyzing a frontal electro-myogram and an electroencephalogram (EEG); and the state entropy can be obtained by the EEG. The two can reflect the excitability of frontal skeletal muscles and the suppression of cerebral cortex in a resuscitation phase. When both of the RE and the SE remain at high level values, it indicates that the person being monitored is awake; when both of the RE and the SE remain at low level values and hemodynamic parameters are stable, it indicates that the person being monitored is at an appropriate level of anesthesia; when the RE rises and the SE remains constant at a relatively low level value, it indicates that the patient may have bodily activity or the patient may feel pain; and when the RE rises and the SE remains unchanged at a relatively high level value, it indicates that the patient may be awake. The RE and the SE can be used to determine the pain threshold and sedation indexes. In addition, the depth of anesthesia index may further include a surgical pleth index (SPI, also called a surgical stress index). It can be used to monitor the number of hemodynamic responses in the patient to surgical stimuli and analgesic treatment during general anesthesia, and can reflect increased sympathetic activity in the patient in response to pain or nociceptive stimuli. In an alternative embodiment, a bispectral index (BIS) may be selected as the depth of anesthesia index. It will be appreciated that the depth of anesthesia index may also be arbitrary in the art and will not be further exemplified.

A combination of the plurality of anesthesia devices can provide a better anesthetic effect, as illustrated by a combination of inhalation anesthesia and injection anesthesia. Inhalation anesthesia takes effect and loses effect rapidly in the induction and recovery periods of anesthesia, but some patients may be unable to withstand physiological responses such as vasodilation caused by inhalation anesthesia for a short period of time. Injection anesthesia is more likely to cause respiratory depression than inhalation anesthesia. The combination of the two can exert their respective advantages to achieve a better anesthetic effect. This places high demands on the anesthetic dosage distribution for inhalation anesthesia and injection anesthesia.

In view of this, the present application provides the anesthesia control system 105. As shown in FIG. 1, the anesthesia control system 105 is connected to the physiological parameter acquisition apparatus 101, the plurality of anesthesia devices (e.g., the first anesthesia device 102 and the second anesthesia device 103) and the depth of anesthesia index acquisition apparatus 104. The connections described above for the anesthesia control system 105 enable communication between various settings. In particular, the physiological parameters of the patient may be acquired from the physiological parameter acquisition apparatus 101; a current anesthetic dosage, such as anesthetic concentration, dosage, injection rate information, may be acquired from the anesthesia devices; and a current depth of anesthesia index of the patient can be acquired from the depth of anesthesia index acquisition apparatus 104. Meanwhile, the anesthesia control system 105 is further provided with a target depth of anesthesia index. The target depth of anesthesia index may be empirically set by a user, such as an anesthetist. In another embodiment, the target depth of anesthesia index may also be automatically set by the anesthesia control system 105, e.g., automatically assigned based on the patient's age, gender, medical history, etc.

The type of the anesthesia control system 105 may be varied, for example, the anesthesia control system 105 may be a controller circuit including a processor. The controller circuit may be configured within a stand-alone control system, and accordingly, the control system may communicate with various other components within the anesthesia control system 105 by wired or wireless means. In addition, the anesthesia control system 105 may be integrated within a certain apparatus in the anesthesia system 100. For example, it may be integrated in the anesthesia device to increase the degree of integration of the instrument. In one example, the anesthesia control system may be disposed at a remote terminal. For example, it may be disposed on a public server of a hospital so that operations with anesthesia in a plurality of operating rooms can be controlled simultaneously. In another example, the anesthesia control system may be disposed in a cloud server in a remote communication manner, such as 5G communication technology.

With the above-described configuration of the present application, the anesthesia control system 105 can comprehensively determine the current physiological conditions of the patient, the device parameters of the plurality of anesthesia devices such as the inhalation anesthesia device and the injection anesthesia device, the depth of anesthesia index of the patient, and the like, thereby providing a comprehensive control scheme while regulating the anesthetic parameters of the plurality of anesthesia devices. Such a control scheme can simultaneously take into account the interactions between different anesthesia devices, e.g., a common sedative and/or analgesic effect between different drugs used by the inhalation and injection anesthesia devices. An antagonistic action of anesthetics between different anesthesia devices may also be considered, e.g., the effect of an analgesic anesthetic on the sedative effect or the effect of a sedative anesthetic on the analgesic effect. In this way, there is neither underdosage of the anesthetic due to the antagonistic action, nor harm to the patient due to excessive use of the total anesthetic. In addition, the anesthesia control system 105 is more secure than human determination and adjustment.

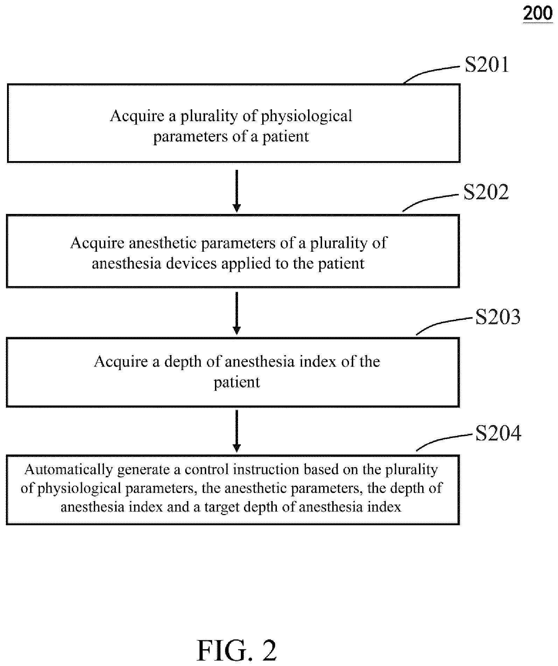

Some embodiments of the present application further provide a control method for an anesthesia control system. The anesthesia system includes a controller circuit used to execute an anesthesia control method. Referring to FIG. 2, FIG. 2 is a control method flowchart of an anesthesia control system according to some embodiments of the present application.

In step 201, a plurality of physiological parameters of a patient are acquired. In this step, the plurality of physiological parameters may be acquired from the physiological parameter acquisition apparatus 101 by the anesthesia control system 105 shown in FIG. 1. In particular, the physiological parameter acquisition apparatus 101 may be connected to the anesthesia control system 105, and may acquire the physiological parameters of the patient 110 in real time and further transmit the physiological parameters to the anesthesia control system 105.

The plurality of physiological parameters may include several among heart rate, oxygen saturation, blood pressure, blood glucose, end-tidal carbon dioxide concentration, and end-tidal oxygen concentration. The inventors have found that the physiological parameters of the patient will have an effect on the appropriate anesthetic dosage and that there are differences between different populations. Thus, the present application provides more comprehensive and reliable analytical data for anesthesia control of the anesthesia control system 105 through the acquisition of the plurality of physiological parameters.

In step 202, anesthetic parameters of a plurality of anesthesia devices applied to the patient are acquired. Referring comprehensively to FIG. 1, the plurality of anesthesia devices may include a first anesthesia device 102 and a second anesthesia device 103.

The first anesthesia device 102 may be an inhalation anesthesia device. Accordingly, an anesthetic thereof may include sevoflurane, desflurane, isoflurane, etc. Further, the anesthetic parameters may be the dosage (e.g., concentration, inhalation rate, etc.) of one or more of the anesthetics described above.

The second anesthesia device 103 may be an injection anesthesia device. Accordingly, an anesthetic thereof may include hexobarbital sodium, methohexital sodium, etc. Further, the anesthetic parameters may be the injection amount (e.g., injection rate, anesthetic concentration, etc.) of one or more of the anesthetics described above.

The anesthesia devices may be self-provided with an anesthetic dosage monitoring module, through which the anesthetic dosage is transmitted to the anesthesia control system.

In step 203, a depth of anesthesia index of the patient is acquired. Referring comprehensively to FIG. 1, the depth of anesthesia index may be acquired from the depth of anesthesia index acquisition apparatus 104. Specifically, the depth of anesthesia index acquisition apparatus 104 may include an EEG device, so that an EEG signal of the patient is acquired in real time, and the EEG signal acquired in real time is analyzed and processed to obtain a desired depth of anesthesia index. The depth of anesthesia index can be evaluated by indexes in the art such as RE, SE and BIS with reference to the above description of the present application, and will not be described in detail here. Further, the depth of anesthesia index is transmitted to the anesthesia control system 105 for integrated processing.

To this end, the anesthesia control system 105 acquires necessary parameters for performing control instruction generation. In step 204, a control instruction is automatically generated based on the plurality of physiological parameters, the anesthetic parameters, the depth of anesthesia index, and a target depth of anesthesia index, the control instruction being used to control the anesthetic parameters of each of the plurality of anesthesia devices such that the depth of anesthesia index of the patient is regulated to the target depth of anesthesia index.

The anesthesia control system 105 enables comprehensive determination through input of the physiological parameters of the patient, the anesthetic parameters of the anesthesia devices, the depth of anesthesia index of the patient, and the target depth of anesthesia index. Anesthetic parameter regulation for each of the plurality of anesthesia devices is also considered. Possible adverse effects of isolated regulation are thus avoided.

It will be appreciated that the above merely schematically illustrates the embodiments of the present application, but the present application is not limited thereto. For example, the order of execution between operations may be appropriately adjusted. In addition, some other operations may be added or some operations may be omitted. Those skilled in the art could make appropriate variations according to the above disclosure, rather than being limited by the above descriptions.

The control instruction can be generated based on a trained learning network. Training and use of the learning network will be exemplarily described below.

The learning network is completed through preparation of training data, selection and construction of a learning network model, and training, testing, and optimization of the learning network.

In some embodiments, the learning network is obtained by training based on a data set of a plurality of parameters of a sample (a known input) and anesthetic parameters (an expected output) corresponding thereto. Specifically, the training of the learning network includes the following step 1 to step 3.

Step 1: acquiring physiological parameters of different patients, anesthetic parameters of anesthesia devices, and depth of anesthesia indexes of the patients in a plurality of successful anesthesia cases, and using the same as a sample parameter set. Further, the parameter set is input into a learning network, and then normalization processing is performed based on a normalization layer in the learning network.

Step 2: acquiring an anesthetic parameter set for each of the successful cases, where the anesthetic parameters include the anesthetic parameters of each of the plurality of anesthesia devices.

Step 3: training the learning network by taking the sample parameter set consisting of the physiological parameters of different patients, the anesthetic parameters of the anesthesia devices, and the depth of anesthesia indexes of the patients as an input and the anesthetic parameter set as an output to obtain the trained learning network. In some embodiments, the trained learning network may be updated based on a new sample set and an anesthetic parameter set corresponding thereto.

In some embodiments, the learning network is trained based on a ResNet (Residual Network) or a VGGNet (Visual Geometry Group Network) or other well-known models. Since the number of processing layers in the ResNet can be set large (as large as 1000 or more), classification (for example, determination of artifact type) based on this network structure can achieve a better effect. In addition, it is easier for the ResNet to optimize the learning network based on more training data.

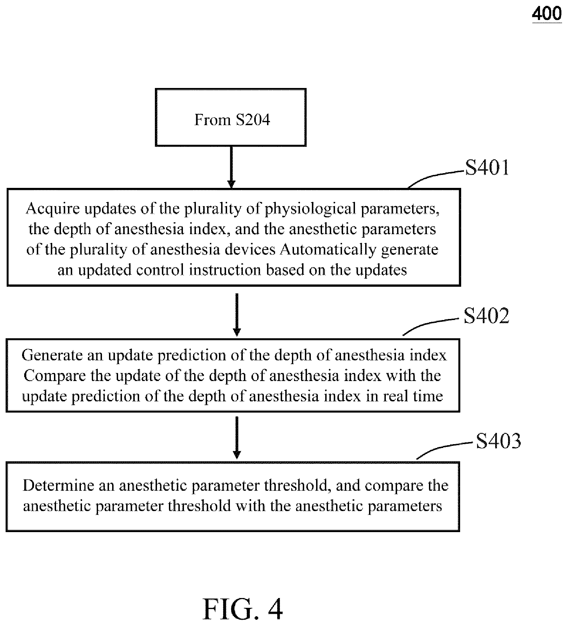

FIG. 4 illustrates a learning network 150 according to some embodiments of the present invention. As shown in FIG. 4, the learning network 150 includes an input layer 151, processing layers (or referred to as hidden layers) 152, and an output layer 153. In some embodiments, as shown in FIG. 4, the processing layers 152 include a first convolutional layer 155, a first pooling layer 156, and a fully-connected layer 157. The first convolutional layer 155 is configured to convolve each of the inputted parameters to obtain a feature map of the first convolutional layer. The first pooling layer 156 pools (downsamples) the feature map of the first convolutional layer to compress the feature map of the first convolutional layer and extract main features thereof, so as to obtain a feature map of the first pooling layer. The fully-connected layer 157 may output a determination result based on the feature map of the first pooling layer.

Although FIG. 4 shows the example of only one convolutional layer, in other examples, there may be any number of convolutional layers, and the number of the convolutional layers may be appropriately adjusted according to the size of input data in the learning network, etc. For example, a second convolutional layer and a second pooling layer (not shown) are further included between the first pooling layer 156 and the fully-connected layer 157, or a second convolutional layer and a second pooling layer, as well as a third convolutional layer and a third pooling layer (not shown), and so on are further included between the first pooling layer 156 and the fully-connected layer 157.

Although FIG. 4 only shows that the convolutional layer is connected to the input layer, the pooling layer is connected to the convolutional layer, and the fully-connected layer is connected to the pooling layer, in other examples, any number of processing layers of any type may be provided between any two of the aforementioned layers. For example, a normalization layer is provided between the convolutional layer and the input layer to normalize the input parameters, or an activation layer is provided between the fully-connected layer and the pooling layer to perform nonlinear mapping on a feature map of the pooling layer using a ReLU (rectified linear unit) activation function.

In some embodiments, each layer includes several neurons 160, and the number of neurons in each layer may be the same or set differently according to needs. The sample data set (a known input) and the anesthetic parameters (an expected output) are input into the learning network, the number of the processing layers in the learning network and the number of neurons in each of the processing layers are set, and the weight and/or bias of the learning network is estimated (or regulated or calibrated), so as to identify the mathematical relationship between the known input and the expected output and/or identify and characterize the mathematical relationship between the input and output of each layer. In the learning process, (part of) input data is usually used, and a network output is created for the input data; then, the created network output corresponding to the known input is compared with the expected output of the data set, and the difference thereof is a loss function; and the loss function is used to iteratively update network parameters (weight and/or bias) to continuously decrease the loss function, so as to train a neural network model with a higher accuracy. In some embodiments, many functions can be used as the loss function, including, but not limited to, mean squared error (mean suqared), cross entropy error, and the like. When the learning network is created or trained completely, a desired anesthetic parameter can be acquired as long as the above-mentioned physiological parameters of the patient, the anesthetic parameters of the anesthesia devices, and the depth of anesthesia index of the patient, which will be used to generate the control signal, are input into the learning network.

In one embodiment, although the configuration of the learning network 150 is guided by dimensions such as priori knowledge, input, and output of an estimation problem, the learning itself is regarded as a "black box," and implements optimal approximation of required output data mainly depending on or exclusively according to input data. In various alternative implementations, clear meaning may be assigned to some data representations in the learning network 150 using some aspects and/or features of data, an imaging geometry, a reconstruction algorithm, or the like. This helps to accelerate training. This creates an opportunity to separately train (or pre-train) or define some layers in the learning network 150.

It is understood that a deep learning method is characterized by the use of one or more network architectures to extract or simulate data of interest. The deep learning method may be implemented using one or a plurality of processing layers (for example, an input layer, an output layer, a convolutional layer, a normalization layer, a sampling layer, or the like, where processing layers of different numbers and functions may exist according to different deep learning network models), where the configuration and number of the layers allow a deep learning network to process complex information extraction and modeling tasks. Specific parameters (also referred to as "weight" or "bias") of the network are usually estimated by means of a so-called learning process (or training process). The learned or trained parameters usually result in (or output) a network corresponding to layers of different levels, so that extraction or simulation of different aspects of initial data or the output of a previous layer usually may represent the hierarchical structure or concatenation of layers. Processing may be performed layer by layer, that is, "simple" features may be correspondingly extracted from input data for an earlier or higher-level layer, and then these simple features are combined into a layer exhibiting features of higher complexity. In practice, each layer (or more specifically, each "neuron" in each layer) may process input data as output data for representation by using one or a plurality of linear and/or non-linear transformations (so-called activation functions). The number of the plurality of "neurons" may be constant among the plurality of layers or may vary from layer to layer.

As discussed herein, as part of initial training of a deep learning process for solving a specific problem, a training data set includes a known input value and an expected (target) output value (e.g., a determination result) finally outputted in the deep learning process. In this manner, a deep learning algorithm can (in a supervised or guided manner or an unsupervised or unguided manner) process the training data set until a mathematical relationship between a known input and an expected output is identified and/or a mathematical relationship between the input and output of each layer is identified and represented. In the learning process, (a part of) input data is usually used, and a network output is created for the input data. Afterwards, the created network output is compared with the expected output of the data set, and then the difference between the created and expected outputs is used to iteratively update network parameters (weight and/or bias). A stochastic gradient descent (SGD) method may usually be used to update network parameters. However, those skilled in the art should understand that other methods known in the art may also be used to update network parameters. Similarly, a separate validation data set may be used to validate a trained learning network, where both a known input and an expected output are known. The known input is provided to the trained learning network so that a network output can be obtained, and then the network output is compared with the (known) expected output to validate prior training and/or prevent excessive training.

The accuracy of anesthesia control during anesthesia is very important. In some embodiments of the present application, some examples for improving the accuracy of control and ensuring the safety of anesthesia are provided. An exemplary description on these measures will be provided below.

Referring to FIG. 4, FIG. 4 is a control method flowchart 400 of an anesthesia control system according to some additional embodiments of the present application. The control method of this example may be performed on the basis of a method flowchart 200, e.g., step 204. A detailed description is provided below.

In step 401, updates of the plurality of physiological parameters, the depth of anesthesia index, and the anesthetic parameters of the plurality of anesthesia devices are acquired; and an updated control instruction is automatically generated based on the updates.

It can be understood that during anesthesia, the physiological parameters, the depth of anesthesia index, and the anesthetic parameters are constantly and dynamically changing. Therefore, the above-described example of the present application discloses that dynamic update of the control instruction is implemented according to the dynamic change of the above-described parameters, thereby enabling more accurate anesthesia. The above process may be implemented by the anesthesia control system 105. The dynamically updated control instruction can more accurately meet the requirements for proper anesthesia, and better adapt to changes in the situation caused by individual accidental factors.

In order to further improve the safety of anesthesia, in step 402, an update prediction of the depth of anesthesia index based on the plurality of physiological parameters, the depth of anesthesia index, the anesthetic parameters and a target depth of anesthesia index is generated; and the update of the depth of anesthesia index is compared with the update prediction of the depth of anesthesia index in real time, and a notification is issued when the two are greater than a certain threshold.

With the above configuration, the anesthesia control system 105 can be used to provide an accurate prediction of abnormal anesthesia. In conventional prediction manners, whether or not a patient is in a safe state is determined only by observed physiological indexes, so that the possibility of an imminent risk of anesthesia cannot be predicted effectively. Meanwhile, the anesthesia control system 105 of the present application may comprehensively predict a future change tendency of the depth of anesthesia index of the patient according to various current physiological parameters, the depth of anesthesia index, the anesthetic parameters, and other factors using artificial intelligence means such as a learning network. This provides conditions for a comparison between an actual depth of anesthesia index and a predicted depth of anesthesia index. When a comparison result shows that the bias between the two is large, it indicates that the risk of anesthesia is high, and at this time, the attention of the doctor can be prompted by means of an alarm or the like, thereby greatly improving the safety of anesthesia. It should be understood that the above threshold may be manually set, or may be autonomously determined by the anesthesia control system 105, which will not be described in detail.

Further, in step 403, an anesthetic parameter threshold is determined, the anesthetic parameter threshold is compared with the anesthetic parameter, and a notification is issued if the anesthetic parameter is greater than the threshold.

The above configuration can further improve the security. Particularly in a fully automated anesthesia process, the control instruction of the anesthesia control system 105 not only focuses on whether the depth of anesthesia index is up to standard, but also can ensure that the dosage of each anesthetic is not excessive based on the depth of anesthesia index being up to standard. In case of an over-dosage, the operator will be notified. Similar to step 402, the threshold in step 403 may be set manually or may be determined autonomously by the anesthesia control system 105.

It will be appreciated that the above merely schematically illustrates the embodiments of the present application, but the present application is not limited thereto. For example, the order of execution between operations may be appropriately adjusted. In addition, some other operations may be added or some operations may be omitted. Those skilled in the art could make appropriate variations according to the above disclosure, rather than being limited by the above descriptions.

Some embodiments of the present application provide the anesthesia control system 105. It will be appreciated that the anesthesia control system 105 may be any system for executing the method described in any embodiment of the present application. As recorded above, the type of the anesthesia control system 105 may be varied, for example, the anesthesia control system 105 may be a controller circuit including a processor. The controller circuit may be configured within a stand-alone control system. In addition, the anesthesia control system 105 may be integrated within a certain apparatus in the anesthesia system 100. For example, it may be integrated in the anesthesia devices. In one example, the anesthesia control system may be disposed at a remote terminal. For example, it may be disposed on a public server of a hospital so that operations with anesthesia in a plurality of operating rooms can be controlled simultaneously. In another example, the anesthesia control system may be disposed in a cloud server in a remote communication manner, such as 5G communication technology.

Some embodiments of the present application further provide a non-transitory computer-readable medium. The non-transitory computer-readable medium stores a computer program having at least one code segment that is executable by a machine to: acquire a plurality of physiological parameters of a patient; acquire anesthetic parameters of a plurality of anesthesia devices applied to the patient; acquire a depth of anesthesia index of the patient; and automatically generate a control instruction based on the plurality of physiological parameters, the anesthetic parameters, the depth of anesthesia index, and a target depth of anesthesia index, the control instruction being used to control the anesthetic parameters of each of the plurality of anesthesia devices such that the depth of anesthesia index of the patient is regulated to the target depth of anesthesia index.

Optionally, the control instruction is automatically generated based on a trained learning network.

Optionally, the anesthetic parameters of the plurality of anesthesia devices include anesthetic parameters of an inhalation anesthesia device and anesthetic parameters of an injection anesthesia device.

Optionally, the depth of anesthesia index includes at least one of a sedation index, a pain threshold index, and a muscle relaxation index.

Optionally, the plurality of physiological parameters include several among heart rate, oxygen saturation, blood pressure, blood glucose, end-tidal carbon dioxide concentration, and end-tidal oxygen concentration.

Optionally, the non-transitory computer-readable medium is further configured to: acquire updates of the plurality of physiological parameters, the depth of anesthesia index, and the anesthetic parameters of the plurality of anesthesia devices; and automatically generate an updated control instruction based on the updates.

Optionally, the non-transitory computer-readable medium is further configured to: generate an update prediction of the depth of anesthesia index based on the plurality of physiological parameters, the depth of anesthesia index, the anesthetic parameters and a target depth of anesthesia index; and compare the update of the depth of anesthesia index with the update prediction of the depth of anesthesia index in real time, and issue a notification when the two are greater than a certain threshold.

Optionally, the non-transitory computer-readable medium is further configured to: determine an anesthetic parameter threshold, compare the anesthetic parameter threshold with the anesthetic parameters, and issue a notification when the anesthetic parameters are greater than the threshold.

The purpose of providing the above specific embodiments is to allow the disclosure of the present application to be understood more thoroughly and comprehensively; however, the present application is not limited to said specific embodiments. A person skilled in the art should understand that various modifications, equivalent replacements, changes and the like can be further made to the present application and should be included in the scope of protection of the present application as long as these changes do not depart from the spirit of the present application.

## Claims

1. An anesthesia control system configured to:
acquire a plurality of physiological parameters of a patient;
acquire anesthetic parameters of a plurality of anesthesia devices applied to the patient;
acquire a depth of anesthesia index of the patient; and
automatically generate a control instruction based on the plurality of physiological parameters, the anesthetic parameters, the depth of anesthesia index, and a target depth of anesthesia index, the control instruction being used to control the anesthetic parameters of each of the plurality of anesthesia devices such that the depth of anesthesia index of the patient is regulated to the target depth of anesthesia index.

2. The anesthesia control system according to claim 1, wherein
the control instruction is automatically generated based on a trained learning network.

3. The anesthesia control system according to claim 1, wherein
the anesthetic parameters of the plurality of anesthesia devices comprise anesthetic parameters of an inhalation anesthesia device and anesthetic parameters of an injection anesthesia device.

4. The anesthesia control system according to claim 1, wherein
the depth of anesthesia index comprises at least one of a sedation index, a pain threshold index, and a muscle relaxation index.

5. The anesthesia control system according to claim 1, wherein
the plurality of physiological parameters comprise several among heart rate, oxygen saturation, blood pressure, blood glucose, end-tidal carbon dioxide concentration, and end-tidal oxygen concentration.

6. The anesthesia control system according to claim 1, further configured to:
acquire updates of the plurality of physiological parameters, the depth of anesthesia index, and the anesthetic parameters of the plurality of anesthesia devices; and
automatically generate an updated control instruction based on the updates.

7. The anesthesia control system according to claim 6, further configured to:
generate an update prediction of the depth of anesthesia index based on the plurality of physiological parameters, the depth of anesthesia index, the anesthetic parameters and a target depth of anesthesia index; and
compare the update of the depth of anesthesia index with the update prediction of the depth of anesthesia index in real time, and issue a notification when the two are greater than a certain threshold.

8. The anesthesia control system according to claim 1, further configured to:
determine an anesthetic parameter threshold, compare the anesthetic parameter threshold with the anesthetic parameters, and issue a notification when the anesthetic parameters are greater than the threshold.

9. An anesthesia system comprising:
a physiological parameter acquisition apparatus used to acquire a plurality of physiological parameters of a patient;
a plurality of anesthesia devices used to administer an anesthetic to the patient;
a depth of anesthesia index acquisition apparatus used to acquire a depth of anesthesia index of the patient; and
the anesthesia control system according to any of claims 1-8, the anesthesia control system being connected to the physiological parameter acquisition apparatus, the plurality of anesthesia devices and the depth of anesthesia index acquisition apparatus.

10. A non-transitory computer-readable medium that stores a computer program having at least one code segment that is executable by a machine to:
acquire a plurality of physiological parameters of a patient;
acquire anesthetic parameters of a plurality of anesthesia devices applied to the patient;
acquire a depth of anesthesia index of the patient; and
automatically generate a control instruction based on the plurality of physiological parameters, the anesthetic parameters, the depth of anesthesia index, and a target depth of anesthesia index, the control instruction being used to control the anesthetic parameters of each of the plurality of anesthesia devices such that the depth of anesthesia index of the patient is regulated to the target depth of anesthesia index.

11. The non-transitory computer-readable medium according to claim 10, wherein
the control instruction is automatically generated based on a trained learning network.

12. The non-transitory computer-readable medium according to claim 10, wherein
the anesthetic parameters of the plurality of anesthesia devices comprise anesthetic parameters of an inhalation anesthesia device and anesthetic parameters of an injection anesthesia device.

13. The non-transitory computer-readable medium according to claim 10, wherein
the depth of anesthesia index comprises at least one of a sedation index, a pain threshold index, and a muscle relaxation index.

14. The non-transitory computer-readable medium according to claim 10, wherein
the plurality of physiological parameters comprise several among heart rate, oxygen saturation, blood pressure, blood glucose, end-tidal carbon dioxide concentration, and end-tidal oxygen concentration.

15. The non-transitory computer-readable medium according to claim 10, further configured to:
acquire updates of the plurality of physiological parameters, the depth of anesthesia index, and the anesthetic parameters of the plurality of anesthesia devices; and
automatically generate an updated control instruction based on the updates.

16. The non-transitory computer-readable medium according to claim 15, further configured to:
generate an update prediction of the depth of anesthesia index based on the plurality of physiological parameters, the depth of anesthesia index, the anesthetic parameters and a target depth of anesthesia index; and
compare the update of the depth of anesthesia index with the update prediction of the depth of anesthesia index in real time, and issue a notification when the two are greater than a certain threshold.

17. The non-transitory computer-readable medium according to claim 10, further configured to:
determine an anesthetic parameter threshold, compare the anesthetic parameter threshold with the anesthetic parameters, and issue a notification when the anesthetic parameters are greater than the threshold.
